# EUROPÄISCHE PATENTANMELDUNG

(11) **EP 1 033 167 A2**
(43) Veröffentlichungstag der Anmeldung: **06.09.2000**
(21) Anmeldenummer: 00102261.5
(22) Anmeldetag: 15.02.2000
(51) Int. Cl.: B01J 8/06, B01J 19/24

(54) **Rohrbündelreaktor mit gestuftem Innendurchmesser**

(30) Priorität: 03.03.1999 DE 19909340
(71) Anmelder: BASF AKTIENGESELLSCHAFT, 67056 Ludwigshafen (DE)
(72) Erfinder: Olbert, Gerhard, 69221 Dossenheim (DE); Corr, Franz, 67067 Ludwigshafen (DE)
(74) Vertreter: Isenbruck, Günter, Dr.

(57) **Zusammenfassung**

Es wird ein Reaktor mit einem Kontaktrohrbündel vorgeschlagen, wobei durch die Kontaktrohre ein fluides Reaktionsgemisch und durch den die Kontaktrohre umgebenden Raum ein Wärmetauschmittel geleitet wird und wobei die Kontaktrohre im Bereich des Reaktionsgemischeintritts gegenüber dem Reaktionsgemischaustritt einen verringerten Innendurchmesser aufweisen. Der Reaktor ist insbesondere zur Durchführung von katalytischen Gasphasenoxidationen, bevorzugt zur Herstellung von Phthalsäureanhydrid, (Meth)acrolein, (Meth)acrylsäure, Malein-säureanhydrid, Oxalsäure und/oder Glyoxal, geeignet.

## Beschreibung

Die Erfindung betrifft einen Reaktor mit einem Kontaktrohrbündel, ein Verfahren zur Herstellung des Reaktors sowie eine Verwendung.

Rohrbündelreaktoren werden in großem Umfang zur Durchführung chemischer Reaktionen, insbesondere von Reaktionen mit großer Wärmetönung, bevorzugt für stark exotherme Gasphasenreaktionen, eingesetzt.

Zur Temperaturregelung sind die Reaktionsrohre von einem Wärmeträgermedium, beispielsweise von einer Salzschmelze, umgeben. Trotz dieser Thermostatisierung kommt es entlang der Katalysatorschüttung zur Ausbildung sogenannter heißer Flecken ( 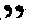 hot spots"), mit erhöhter Temperatur gegenüber der übrigen Katalysatorschüttung. Dies mindert einerseits in diesem Kontaktrohrabschnitt die Lebensdauer des Katalysators und beeinträchtigt andererseits die Selektivität der chemischen Reaktion.

Verschiedene Gegenmaßnahmen zur Überwindung des genannten Nachteil werden im Stand der Technik bereits empfohlen und sind beispielsweise in DE-A-44 31 949 beschrieben. Ein Vorschlag besteht in der Verkleinerung des Durchmessers der Kontaktrohre, um so die Wärmeableitung je Volumeneinheit des Katalysators zu erhöhen. Nachteilig an dieser Methode ist jedoch, daß sie für eine bestimmte Produktionsleistung erforderliche Anzahl katalysatorgefüllter Kontaktrohre in notwendiger Weise erhöht, was sowohl die Fertigungskosten des Reaktors als auch die zum Füllen und Entleeren der Kontaktrohre im Katalysator erforderliche Zeitdauer steigert. In DE-A-44 31 949 wird eine Reduzierung der Heißpunkttemperaturen in den Rohren eines Rohrreaktors mit Wärmetauschmittelkreislauf im die Kontaktrohre umgebenen Raum durch eine mäanderförmige Führung des Wärmetauschmittels in Gleichstrom mit den Reaktionsgasen sowie durch Einhaltung bestimmter, geringer Temperaturdifferenzen des Wärmetauschmittels von der Eintrittsstelle bis zur Austrittsstelle aus dem Reaktor offenbart.

Nach einem anderen vorgeschlagenen Verfahren wird die Ausbildung der Heißpunkte dadurch zu unterdrücken versucht, daß man die volumenspezifische Aktivität der katalytischen Beschickung längs der Kontaktrohre variiert. Diese Verfahrensweise erfordert jedoch die Anwendung entweder mindestens zweier Katalysatoren unterschiedlicher Aktivität oder die Mitverwendung von Inertmaterial. Außerdem verkompliziert diese Verfahrensweise das Füllen der Kontaktrohre.

Es ist Aufgabe der Erfindung, einen Reaktor mit einem Kontaktrohrbündel zur Verfügung zu stellen, der in der Weise ausgestaltet ist, daß bei der Durchführung von exothermen Reaktionen in den Rohren des Kontaktrohrbündels das Auftreten von 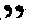 hot spots" überwiegend oder vollständig vennieden wird. Diese Aufgabe soll in einfacher, wirtschaftlicher Weise, durch eine geeignete Ausbildung der Reaktionsrohre, gelöst werden. Es ist weiterhin Aufgabe der Erfindung, ein Verfahren zur Herstellung eines derartigen Reaktors mit einem Kontaktrohrbündel zu Verfügung zu stellen.

Die Lösung geht aus von einem Reaktor mit einem Kontaktrohrbündel, wobei durch die Kontaktrohre ein fluides Reaktionsgemisch und durch den die Kontaktrohre umgebenden Raum ein Wärmetauschmittel geleitet wird. Die Lösung ist dadurch gekennzeichnet, daß die Kontaktrohre im Bereich des Reaktionsgemischeintritts gegenüber dem Reaktionsgemischaustritt einen verringerten Innendurchmesser aufweisen.

Es wurde überraschend gefunden, daß die Verringerung des Rohrinnendurchmessers im Bereich des Reaktionsgemischeintritts einen erheblichen Einfluß auf die Absenkung von katalysatorschädigenden Temperaturspitzen hat, so daß das Auftreten von 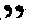 hot spots" überwiegend oder vollständig vermieden wird.

Die Baugröße der Reaktoren, die gemäß der Erfindung ausgebildet sein können, ist grundsätzlich nicht eingeschränkt. Anwendungstechnisch zweckmäßig werden große Reaktoren angesetzt, in denen Bündel aus einer Vielzahl von Kontaktrohren, bevorzugt über 10.000, häufig im Bereich von 15.000 bis 30.000, angeordnet werden können. Innerhalb des Reaktors sind die Kontaktrohre im Normalfall regelmäßig verteilt angeordnet (vergleiche DE-A-44 31 949). Die Kontaktrohre, die gegebenenfalls geträgerte Katalysatoren enthalten können, sind mit ihren Enden in Rohrböden abdichtend befestigt und münden jeweils am oberen bzw. unteren Ende in eine mit dem Reaktor verbundene Haube. Über diese Hauben wird das die Kontaktrohre durchströmende fluide, d.h. gasförmige oder flüssige Reaktionsgemisch zu- bzw. abgeführt. Durch den die Kontaktrohre umgebenen Raum wird ein Wärmetauschmittelkreislauf geleitet, wobei als Wärmetauschmittel insbesondere fluide Temperaturmedien, häufig Salzschmelzen oder Metallschmelzen, geleitet werden.

Üblicherweise sind die Kontaktrohre aus ferritischem Stahl gefertigt und weisen in typischer Weise eine Wanddicke von ein bis drei Millimeter auf. Ihr Innendurchmesser beträgt in der Regel 20 bis 35 Millimeter, bevorzugt 25 +/- 2 Millimeter. Die Rohrlänge erstreckt sich im Normalfall auf wenige Meter, typischerweise im Bereich von zwei bis vier Meter.

Erfindungsgemäß wird der Innendurchmesser der Kontaktrohre im Bereich des Reaktionsgemischeintritts gegenüber dem Reaktionsgemischaustritt um 5% bis 25%, bevorzugt um 10% bis 15%, verringert. Der Rohrinnendurchmesser wird somit im Bereich des Reaktionsgemischeintritts typischerweise um etwa ein bis fünf Millimeter, bevorzugt um etwa zwei bis drei mm, reduziert.

In bevorzugter Weise wird der Innendurchmesser im Bereich des Reaktionsgemischeintritts auf einer Länge von 1/3 bis zur Hälfte der Gesamtrohrlänge verringert.

In bevorzugter Weise sind die Kontaktrohre erfindungsgemäß so ausgebildet, daß die Rohrinnenwand im Bereich des Übergangs vom geringeren zum größeren Innendurchmesser angeschrägt ist.

Erfindungsgemäße Reaktoren können grundsätzlich jede beliebige Bauform aufweisen, bevorzugt sind sie jedoch zylindrisch ausgebildet. Es sind jedoch auch andere Bauformen möglich, beispielsweise Bauformen mit rechteckigem Querschnitt.

Die Verfahrensaufgabe kann dadurch gelöst werden, daß Rohre mit gleichem Außendurchmesser, jedoch mit größerer Wandstärke für den Bereich am Reaktionsgemischeintritt gegenüber dem Bereich am Reaktionsgemischaustritt eingesetzt werden. Ein derartiges Verfahren wird insbesondere bei der Neukonstruktion von Reaktoren mit Kontaktrohrbündeln zur Anwendung kommen.

Alternativ kann, insbesondere bei bereits vorhandenen Reaktoren, ein Verfahren eingesetzt werden, wonach in ein Kontaktrobrbündel mit konstantem Innendurchmesser der Kontaktrohre in den Bereich am Reaktionsgemischeintritt Inlet-Rohre eingeschoben werden. Als Inlet-Rohre werden bevorzugt Rohre mit einer Wandstärke im Bereich von 1 bis 2,5 Millimeter, bevorzugt von 1 bis 1,5 mm, eingesetzt. Das Material der Inlet-Rohre ist bevorzugt dasselbe, wie das Material der Kontaktrohre.

In verfahrenstechnisch vorteilhafter Weise können die Inlet-Rohre an die Innenwände der Kontaktrohre hydraulisch angepreßt werden. Durch diese Maßnahme können vorgegebene Innendurchmesser exakt eingestellt werden.

Erfindungsgemäß weisen im allgemeinen alle Kontaktrohre eines Kontaktrohrbündels einen im Bereich des Reaktionsgemischeintritts gegenüber dem Reaktionsgemischaustritt verringerten Innendurchmesser auf Die erfindungsgemäße Aufgabe wird jedoch auch dadurch gelöst, daß zumindest der überwiegende Teil der Kontaktrohre im Bereich des Reaktionsgemischeintritts einen verringerten Innendurchmesser aufweist, wobei bevorzugt die im äußeren Reaktorbereich liegenden Kontaktrohre im Eingangsbereich in ihrem Innendurchmesser verringert sind.

Erfindungsgemäße Reaktoren eignen sich besonders zur Durchführung von exothermen Reaktionen, insbesondere katalytischen (Gasphasenoxidationen, bevorzugt zur Herstellung von Phthalsäureanhydrid, (Meth)acrolein, (Meth)acrylsäure, Maleinsäureanhydrid, Oxalsäure und/oder Glyoxal.

Durch die erfindungsgemäße Maßnahme wird eine zuverlässige Absenkung der 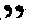 hot spot"-Temperatur erreicht. Die Strömungsgeschwindigkeit des fluiden Reaktionsgemisches wird im Bereich des Reaktionsgemischeintritts erhöht.

Erfindungsgemäße Reaktoren haben weiterhin den Vorteil, daß sie zur Durchführung von chemischen Reaktionen mit besserer Ausbeute, weniger Verbrennung, weniger Nebenreaktionen und weniger Eduktverlust eingesetzt werden können.

Die Erfindung wird im folgenden anhand eines Ausführungsbeispiels näher erläutert:

In einem Verfahren zur großtechnischen Herstellung von Acrylsäure durch katalytische Gasphasenoxidation in einem zylindrischen Rohrbündelreaktor mit ca. 30000 Rohen mit einer Länge von jeweils 3,20 m wurde durch Verengung des Innendurchmessers sämtlicher Rohre jeweils über die gesamte Rohrlänge eine Absenkung der 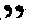 hot spot"- Temperatur von 420°C auf 412°C erreicht und somit in einen Bereich, in dem keine nennenswerte Katalysatorschädigung auftritt, abgesenkt. Eine Verringerung der Innendurchmesser sämtlicher Rohre des Kontaktrohrbündels führte somit zu einer Absenkung der 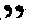 hot spot"- Temperatur um 8°C.

## Patentansprüche

1. Reaktor mit einem Kontaktrohrbündel, wobei durch die Kontaktrohre ein fluides Reaktionsgemisch und durch den die Kontaktrohre umgebenden Raum ein Wärmetauschmittel geleitet wird, dadurch gekennzeichnet, daß die Kontaktrohre im Bereich des Reaktionsgemischeintritts gegenüber dem Reaktionsgemischaustritt einen verringerten Innendurchmesser aufweisen.

2. Reaktor nach Anspruch 1, dadurch gekennzeichnet, daß der Innendurchmesser im Bereich des Reaktionsgemischeintritts gegenüber dem Reaktionsgemischaustritt um 5 bis 25 %, bevorzugt um 10 bis 15%, verringert ist.

3. Reaktor nach Anspruch 1 oder 2, dadurch gekennzeichnet, daß der Innendurchmesser im Bereich des Reaktionsgemischeintritts auf einer Länge von 1/3 bis zur Hälfte der Gesamtrohrlänge verringert ist.

4. Reaktor nach einem der Ansprüche 1 bis 3, dadurch gekennzeichnet, daß die Rohrinnenwand im Bereich des Übergangs vom geringeren zum größeren Innendurchmesser angeschrägt ist.

5. Reaktor nach einem der Ansprüche 1 bis 4, dadurch gekennzeichnet, daß er eine zylindrische Bauform aufweist.

6. Verfahren zur Herstellung eines Reaktors nach einem der Ansprüche 1 bis 5, dadurch gekennzeichnet, daß Rohre mit konstantem Außendurchmesser, jedoch mit größerer Wandstärke für den Bereich am Reaktionsgemischeintritt gegenüber dem Bereich am Reaktionsgemischaustritt eingesetzt werden.

7. Verfahren zur Herstellung eines Reaktors nach einem der Ansprüche 1 bis 5, dadurch gekennzeichnet, daß in ein Kontaktrohrbündel mit konstantem Innendurchmesser der Kontaktrohre in den Bereich am Reaktionsgemischeintritt Inlet-Rohre eingeschoben werden.

8. Verfahren nach Anspruch 7, dadurch gekennzeichnet, daß die Inlet-Rohre hydraulisch angepreßt werden.

9. Verwendung eines Reaktors nach einem der Ansprüche 1 bis 5, oder nach einem der Verfahrensansprüche 6 bis 8 hergestellten Reaktors zur Durchführung von exothermen Reaktionen, insbesondere katalytischen Gasphasenoxidationen, bevorzugt zur Herstellung von Phthalsäureanhydrid, (Meth)acrolein, (Meth)acrylsäure, Malein-säureanhydrid, Oxalsäure und/oder Glyoxal.
